# EUROPEAN PATENT APPLICATION

(11) **EP 2 263 701 A1**
(43) Date of publication of application: **22.12.2010**
(21) Application number: 09162859.4
(22) Date of filing: 16.06.2009
(51) Int. Cl.: A61L 9/03, A01M 1/20

(54) **Apparatus for dispensing a fragrance**

(71) Applicant: Spectrum Designs Ltd, Kowloon Hong Kong (HK)
(72) Inventor: Lui, Edmond, Ap Lee Chau Hong Kong (CN); Muirhead, Alex, Ap Lee Chau Hong Kong (CN)
(74) Representative: Howell, Matthew Dafydd

(57) **Abstract**

Apparatus (40) for dispensing a fragrance, the apparatus (40) comprising a base part (50) which is adapted to be received in an opening of a vessel (20) and a lid part (70) having an outlet (76) permitting the egress of a fragrance, the lid part (70) being adapted to cooperate with the base part (50) to form a cavity therebetween in which an insert (90) bearing the fragrance can be received.

## Description

### Technical Field

The present invention relates to an apparatus for dispensing a fragrance, to an insert for use in such an apparatus and to a lamp having an apparatus for dispensing a fragrance.

### Background to the Invention

In recent years fragrance dispensers for imparting a pleasant fragrance to domestic environments, as well as commercial venues such as spas, hotels and retail outlets, have become popular. A wide variety of such products are available, including aerosol sprays, plug-in fragrance dispensers which are received in a mains electricity outlet and release a fragrance on a continuous basis or at intervals, and gel- and liquid-based systems in which a fragranced gel or liquid contained in a housing is exposed to the air, causing a fragrance to be released into the surrounding environment.

Fragrance products like those described above tend to be functional items which are not particularly aesthetically pleasing, making users wish to conceal them from view. Indeed, in the case of plug-in fragrance dispensers, because of the usual positioning of electrical outlets in domestic residences, even if the dispenser were aesthetically pleasing it would be difficult to display it in a prominent position.

Other types of fragrance dispensers are also available, which heat fragranced oils to release fragranced vapours. Such fragrance dispensers typically comprise a reservoir for holding the oil to be heated positioned above a candle, tea-light or similar heat source, such that the oil is heated by the heat source to release the fragranced vapour through the outlets.

Whilst fragrance dispensers of this type are typically more attractive than the products discussed above, a disadvantage of such arrangements is that they must be refilled with fragranced oils regularly. This is a time-consuming task which can be messy, as the fragranced oils may stain garments, tablecloths and the like. Moreover, the fragranced oils used in dispensers of this type may be flammable, so care must be taken in handling them to minimise the risk of fire. Additionally, the fragranced oils often leave a residue in the reservoirs, such that if it is desired to use an alternative fragrance the reservoir must be thoroughly cleaned, or else a fresh dispenser must be used.

Fragranced candles are also widely used. While they can be pleasing in appearance, they have the disadvantages that they are a potential fire hazard as the flame is unprotected and that they create a pool of hot liquid wax that can cause harm to users and damage to furnishings if accidentally knocked over.

The applicant has realised that a need exists for a fragrance dispenser which has a decorative and aesthetically pleasing appearance, is safe to use when used according to instructions and is easy and convenient to refill with an existing fragrance or with a new fragrance.

### Summary of Invention

According to a first aspect of the present invention there is provided apparatus for dispensing a fragrance, the apparatus comprising a base part which is adapted to be received in an opening of a vessel and a lid part having an outlet permitting the egress of a fragrance, the lid part being adapted to engage with the base part to form a cavity therebetween in which an insert bearing the fragrance can be received.

In use of the apparatus according to the first aspect of the invention, the base part is received in an opening of a vessel containing a heat source such as a liquid wax burner, candle, tea-light or the like and an insert bearing the fragrance is placed on an upper surface of the base part. The lid is then placed on the base part. The insert bearing the fragrance is gently heated by the heat source, and emits the fragrance as a vapour, which passes through the outlet in the lid to the exterior of the apparatus.

This arrangement is very easy to use and avoids the inconvenience of prior art systems. By providing the fragrance as an insert to the apparatus, the apparatus can easily and quickly be replenished with fragrance, or the fragrance can be changed, without having to handle fragranced oils and the like.

The base part may comprises a lip for engagement with the opening of the vessel.

Preferably, the lip extends continuously around a peripheral edge of the base part.

Alternatively, the lip may comprises a plurality of tabs which extend radially outwardly of a peripheral wall of the base part.

The lid part may have an outer dimension which is greater than an outer dimension of the base part such that the lid part is able to engage with the base part by placing the lid part over the base part.

The base part may comprise a retaining formation for retaining the insert in place.

The insert may be formed from a porous material which is impregnated, coated or otherwise provided with the fragrance.

According to a second aspect of the present invention there is provided an insert for an apparatus according to the first aspect of the invention.

According to a third aspect of the invention there is provided a lamp comprising a vessel in which a light and heat source can be received and an apparatus according to the first aspect of the invention.

### Brief Description of the Drawings

Embodiments of the invention will now be described, strictly by way of example only, with reference to the accompanying drawings, of which:
Figure 1 is an exploded schematic cross-sectional view showing a lamp arrangement with a fragrance dispenser;
Figure 2 is a schematic view from above of a base part of the fragrance dispenser shown in Figure 1;
Figure 3 is a schematic view from above of a lid of the fragrance dispenser shown in Figure 1; and
Figure 4 is a schematic view from above of an insert for the fragrance dispenser shown in Figure 1.

### Description of the Embodiments

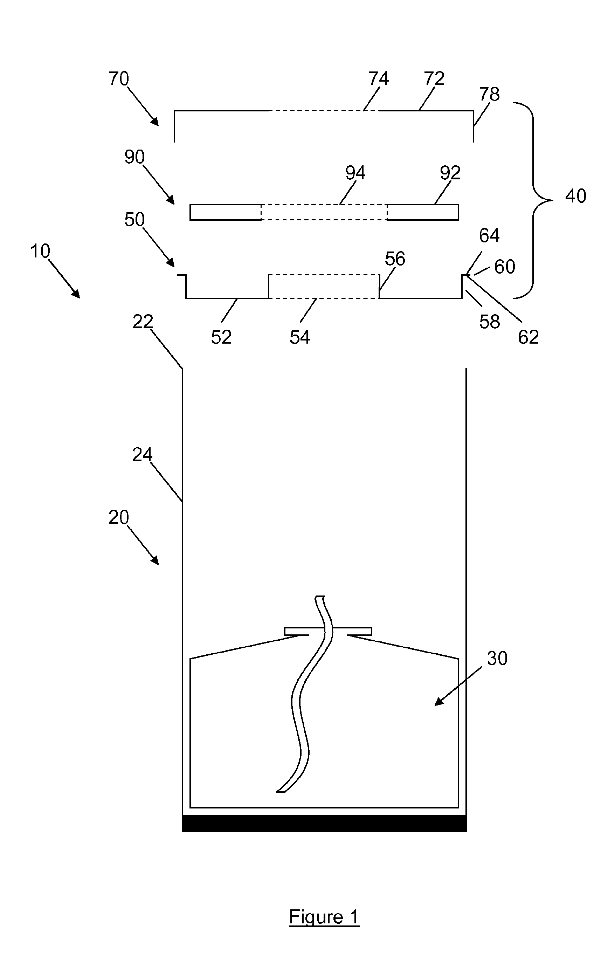

Referring first to Figure 1, a lamp arrangement with a fragrance dispenser is shown generally at 10. The lamp arrangement 10 comprises an open-ended vessel 20 which in this example is made of a transparent, semi-transparent or translucent material such as glass. A light and heat source 30 is received in the vessel 20. In this example the light and heat source 30 is a burner for liquid wax, but it may take other forms, such as a candle, a tea-light or an electric light bulb.

The fragrance dispenser is shown generally at 40 and comprises a base part 50, a lid part 70 and an fragrance-carrying insert 90. In use of the lamp arrangement 10, the base part 50 of the fragrance dispenser 40 is received in the open end of the vessel 20. The lid part 70 engages with the base part 50 to form a cavity in which the insert 90 is received. As the base part 50 is gently heated by the heat emitted by the light source 30, the insert 90 is also heated, causing it to release its fragrance through the lid part 70.

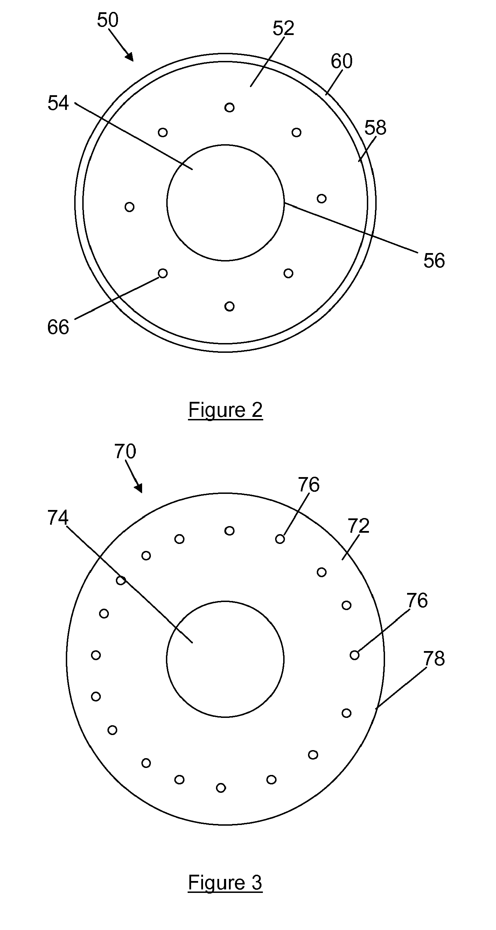

The base part 50 will now be described in more detail with reference to Figures 1 and 2. The base part 50 comprises a tray having as its base a generally circular disc 52 of a heat-conducting material such as aluminium, stainless steel or a ceramic material. It will of course be appreciated that any appropriate heat-conducting material could be used to form the disc 52 of the base part 50. The disc 52 is formed with a generally circular central aperture 54 through which heat from the light and heat source 30 can pass in use of the lamp arrangement 10. A wall 56 surrounds the aperture 54 and extends axially outwardly of the disc 52 (in this example upwardly of the disc 52), to form a retaining formation for the insert 90, as will be described in more detail below. The disc 52 may be solid, or may be have apertures 66 through which heat from the light and heat source 30 may pass to facilitate the process of heating the insert 90.

A peripheral wall 58 extends axially outwardly of the disc 52 (in this example upwardly of the disc 52), and has a lip 60 which extends radially outwardly of the peripheral wall 58. In this example the lip 60 is continuous, that is to say it extends outwardly of the full circumference of the peripheral wall 58, but it will be appreciated that the lip 60 need not be continuous, but may be provided as a plurality of tabs extending radially outwardly of the peripheral wall 58.

In use of the fragrance dispenser 40 the base part 50 is received in the open end of the vessel 20 by the engagement of a lower surface 62 of the lip 60 with an upper edge 22 of a wall 24 of the vessel 20. It will be appreciated that the continuous lip 60 of the example shown in Figures 1 and 2 provides a stable engagement of the base part 50 with the vessel 20, as the lip 60 is in contact with the edge 22 around the entire circumference of the wall 24. Nevertheless, an acceptable engagement of the base part 50 with the vessel 20 can be achieved using a discontinuous lip 60, for example where two or more tabs extend radially outwardly of the peripheral wall 58.

The lid part 70 will now be described in more detail, with reference to Figures 1 and 3. The lid part comprises a generally circular disc 72 which is provided with a generally circular central aperture 74 through which heat from the light and heat source 30 may pass in use of the lamp arrangement 10. The disc 72 is provided with a plurality of outlet perforations 76, which permit the egress of fragrance from the insert 90 in use of the fragrance dispenser 40. In this example the perforations 76 are shown as being generally circular, but it will be appreciated that they can take any form which permits the egress of fragrance.

A peripheral skirt 78 extends axially (in this example downwardly) from an outer edge of the disc 72. In this example the outer diameter of the disc 72 is slightly large than the outer diameter of the lip 60 of the base part 50, such that the lid part 70 can engage with the base part 50 by placing the lid part 70 over the base part 50 such that a lower side 50 of the disc 72 rests on an upper surface 64 of the lip 60 of the base part 50.

When the lid part 70 is engaged with the base part 50 in this way a cavity is formed between the lid part 70 and the base part 50, in which the insert 90 can be received. In this example the peripheral skirt 78 is slightly longer than the height of the wall 58 of the base part 50. This arrangement provides an aesthetically pleasing "seamless" appearance, as the skirt 78 extends beyond the lip 60 and the level of the disc 52 of the base part when the fragrance dispenser is in use and the base part 50 is engaged with the vessel 20, thereby concealing the lip 60 and the disc 52 of the base part. This is particularly advantageous where the vessel 20 is made of a transparent, semi-transparent or translucent material.

In an alternative embodiment, the disc 72 may have a diameter slightly smaller than the inner diameter of the lip 60, such that the skirt 78 can be received within the peripheral wall 58 of the base part 50. This arrangement may offer improved security, in the sense that there is less likelihood that the lid part 70 could accidentally disengage from the base part 50 because of the increased contact and frictional engagement between an inner surface of the wall 58 and the skirt 78, although of course this means that the lid part 70 is more difficult to disengage from the base part 50 for legitimate purposes such as inserting or replacing an insert 90.

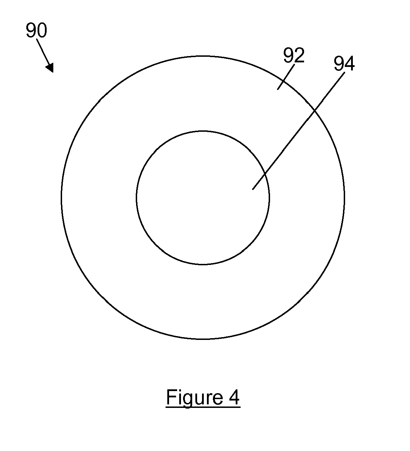

The insert 90 will now be described in more detail with reference to Figures 1 and 4. The insert 90 in this example comprises a generally circular disc 92 having a generally circular central aperture 94. The insert 90 has an outer diameter which is slightly smaller than the outer diameter of the disc 52 of the base part, so that the insert can be received in the cavity formed when the base part 50 engages with the lid part 70 without coming into contact with the peripheral wall 58 of the base part or with the skirt 78 of the lid part. The central aperture 94 has a diameter slightly greater than that of the central aperture 54, such that when the insert 90 is positioned on the base part 50 the wall 56 extends through the central aperture 94, thereby helping to retain the insert 90 in position.

In one embodiment the diameter of the central aperture 94 is such that the insert 90 frictionally engages with the wall 56 of the base part 50 so as to retain the insert 90 in position. However, in this embodiment removing and replacing the insert 90 may be difficult, and slight deviations from nominal diameters of the central aperture 94 of the insert resulting from the manufacturing process may render the insert 90 useless or at least difficult to use effectively. For these reasons it is preferred that the diameter of the central aperture 94 of the insert 90 is such as to allow a degree of free play between the insert 90 and the wall 56 of the base part 50.

The insert 90 is formed of a porous material such cardboard, felt or another fabric material and is impregnated, coated or otherwise provided with a fragrance which will vaporise when the insert is gently warmed.

In use of the lamp arrangement 10, the base part 50 is placed on the open end of the vessel 20, with the lower side 62 of the lip 60 in engagement with the upper edge 22 of the wall 24. The insert 90 is placed on the base part 50 and the lid part 70 is placed on the base part 50. These steps can be performed whilst the light and heat source 30 is alight, or alternatively the light and heat source 30 can be lit using a taper or similar device through the chimney formed by the aligned apertures 54, 94, 74 of the base part 50, the insert 90 and the lid part 70.

The light and heat source 30 gently warms the base part 50, which conducts heat to the insert 90, causing the fragrance in the insert 90 to vaporise. This vaporised fragrance escapes through the perforations 76 of the lid part 70 (and through the central aperture 74) into the environment surrounding the lamp arrangement 10. Where the vessel 20 is transparent, semi transparent or translucent, a pleasant flickering glow from the light and heat source 30 may also be produced.

When the insert 90 is exhausted, or if an alternative fragrance is desired, it is a simple matter to replace the insert 90, requiring only that the lid part 70 be removed. This avoids the difficulties associated with existing oil-based fragrance dispensers of using and storing messy and potentially flammable fragranced oils.

Moreover, a lamp equipped with a fragrance dispenser of the present invention provides an aesthetically pleasing decorative item which performs the dual functions of creating a warm and pleasant flickering glow whilst at the same time dispensing a fragrance into the surrounding environment, which can contribute greatly to creating a comfortable and welcoming ambience in a room.

Where a mixture of fragrances is desired, two or more inserts 90 may be placed on the base part 50 in a stacked configuration. In this way, each of the inserts 90 is heated by the light and heat source 30 and releases its fragrance. Additionally, the or each insert 90 may be provided with more than one fragrance. Thus, subtle and complex fragrances and mixtures of fragrances can be dispensed by the lamp arrangement 10.

Although the components of the exemplary fragrance dispenser 40 described above are said to be generally circular, it will be appreciated that the fragrance dispenser 40 and its component parts may take many forms, so as to fit different shapes of vessel 20.

Moreover, it will be appreciated that the embodiment described above with reference to the drawings is merely one example of a fragrance dispenser, and that other embodiments are possible. For example, where a smoke-free light and heat source such as an electric light bulb is used, the central apertures 54, 74, 94 of the base part 50, lid part 70 and insert 90 respectively are not required, and thus may be omitted.

## Claims

1. Apparatus (40) for dispensing a fragrance, the apparatus (40) comprising a base part (50) which is adapted to be received in an opening of a vessel (20) and a lid part (70) having an outlet (76) permitting the egress of a fragrance, the lid part (70) being adapted to cooperate with the base part (50) to form a cavity therebetween in which an insert (90) bearing the fragrance can be received.

2. Apparatus (40) according to claim 1 wherein the base part (50) comprises a lip (60) for engagement with the opening of the vessel (20).

3. Apparatus (40) according to claim 2 wherein the lip (60) extends continuously around a peripheral edge of the base part (50).

4. Apparatus (40) according to any one of claims 1 to 3 wherein the lip (60) comprises a plurality of tabs which extend radially outwardly of a peripheral wall (58) of the base part (50).

5. Apparatus (40) according to any one of the preceding claims wherein the lid part (70) has an outer dimension which is greater than an outer dimension of the base part (50) such that the lid part (70) is able to engage with the base part (50) by placing the lid part (70) over the base part (50).

6. Apparatus (40) according to any one of the preceding claims wherein the base part (50) comprises a retaining formation (56) for retaining the insert in place.

7. Apparatus (40) according to any one of the preceding claims wherein the insert is formed from a porous material which is impregnated, coated or otherwise provided with the fragrance.

8. An insert (90) for use in an apparatus (40) according to any one of the preceding claims.

9. A lamp (10) comprising a vessel (20) in which a light and heat source (30) can be received and an apparatus (40) according to any one of claims 1 to 7.
